# EUROPÄISCHE PATENTSCHRIFT

(11) **EP 2 201 139 B1**
(45) Veröffentlichungstag und Bekanntmachung des Hinweises auf die Patenterteilung: **07.09.2011**
(21) Anmeldenummer: 08839398.8
(22) Anmeldetag: 10.10.2008
(51) Int. Cl.: C12Q 1/68

(54) **NACHWEIS VON MIT PARODONTITIS ASSOZIIERTEN KEIMEN**
DETECTION OF GERMS ASSOCIATED WITH PERIODONTITIS
DÉTECTION DE GERMES ASSOCIÉS À LA PARODONTITE

(30) Priorität: 10.10.2007 AT 16172007
(43) Veröffentlichungstag der Anmeldung: 30.06.2010
(73) Patentinhaber: Greiner Bio-One GmbH, 72636 Frickenhausen (DE)
(72) Erfinder: WINKLEHNER, Peter, Johannes, 4261 Rainbach (AT); MITTERMAYR, Christian, Rudolf, 4111 Walding (AT)
(74) Vertreter: Chmilewsky-Lehner, Robert
(86) Internationale Anmeldenummer: PCT/EP2008/008580
(87) Internationale Veröffentlichungsnummer: WO 2009/049833

(56) Entgegenhaltungen:
- WO-A-89/06704
- WO-A-02/064824
- WO-A-03/014382
- WO-A-2007/056680
- VIANNA M E ET AL: "Microarrays complement culture methods for identification of bacteria in endodontic infections." ORAL MICROBIOLOGY AND IMMUNOLOGY AUG 2005, Bd. 20, Nr. 4, August 2005 (2005-08), Seiten 253-258, XP002511641 ISSN: 0902-0055
- SAKAMOTO MITSUO ET AL: "Molecular analysis of human oral microbiota" JOURNAL OF PERIODONTAL RESEARCH, Bd. 40, Nr. 3, Juni 2005 (2005-06), Seiten 277-285, XP002511642 ISSN: 0022-3484

### SEQUENZPROTOKOLL

<110> Greiner Bio - One GmbH
   Maybachstrasse 2
   D 72636 Frickenhausen
   DEUTSCHLAND
<120> Nachweis von mit Parodontitis assoziierten Keimen
<160> 8
<210> 1
   <211> 27
   <212> DNA
   <213> künstliche Sequenz
<220>
   <223> Beschreibung der künstlichen Sequenz: Oligonukleotid
<400> 1
   aagaaagctc tcactctccg tcgtcta 27
<210> 2
   <211> 33
   <212> DNA
   <213> künstliche Sequenz
<220>
   <223> Beschreibung der künstlichen Sequenz: Oligonukleotid
<400> 2
   cgctgtggaa gcttgacggt atatcgcaaa ctc 33
<210> 3
   <211> 29
   <212> DNA
   <213> künstliche Sequenz
<220>
   <223> Beschreibung der künstlichen Sequenz: Oligonukleotid
<400> 3
   agtcaacatc tctgtatcct gcgtctgca 29
<210> 4
   <211> 32
   <212> DNA
   <213> künstliche Sequenz
<220>
   <223> Beschreibung der künstlichen Sequenz: Oligonukleotid
<400> 4
   aagagccgta ttgctacgct gccatatctc ta 32
<210> 5
   <211> 31
   <212> DNA
   <213> künstliche Sequenz
<220>
   <223> Beschreibung der künstlichen Sequenz: Oligonukleotid
<400> 5
   gtctcaaagc tccctaaggc tcaaacccat c 31
<210> 6
   <211> 32
   <212> DNA
   <213> künstliche Sequenz
<220>
   <223> Beschreibung der künstlichen Sequenz: Oligonukleotid
<400> 6
   catcgaatta aaccacatgy tccwccgctt gt 32
<210> 7
   <211> 32
   <212> DNA
   <213> künstliche Sequenz
<220>
   <223> Beschreibung der künstlichen Sequenz: Oligonukleotid
<400> 7
   acaagcggwg garcatgtgg tttaattcga tg 37
<210> 8
   <211> 30
   <212> DNA
   <213> künstliche Sequenz
<220>
   <223> Beschreibung der künstlichen Sequenz: Oligonukleotid
<400> 8
   cccgtcaatt cmtttgagtt tyamcsttgc 30

## Beschreibung

Die vorliegende Erfindung beschreibt ein Verfahren, eine mikrofluidische Vorrichtung, sowie einen Kit zum Nachweis und/oder zur Bestimmung von mit Parodontitis assoziierten Bakterien aus einer biologischen Probe.

Parodontitis ist eine bakteriell bedingte Entzündung, die sich in einer weitgehend irreversiblen Zerstörung des Zahnhalteapparats (Parodontium) zeigt.

Die Parodontitis wird durch bakterielle Plaque (Zahnbelag) ausgelöst, einem zäh anhaftenden Biofilm. Hauptunterscheidungsmerkmal ist der bei der Parodontitis vorhandene röntgenologisch nachweisbare Knochenabbau, während die vertieften Zahnfleischtaschen bei einer Gingivitis durch die entzündliche Schwellung der Gingiva zustande kommen. Sowohl bei der Gingivitis als auch bei der Parodontitis werden aus dem Biofilm bakterielle Stoffwechsel- und Zerfallsprodukte freigesetzt, die Abwehrreaktionen des Körpers auslösen. Die Hauptrolle bei der Gewebszerstörung selbst spielt das eigene Immunsystem, das versucht, die Bakterien zu beseitigen. Diese Immunabwehr besteht aus einer vielfältigen Abfolge von Reaktionen und Aktionen, bei der verschiedene Entzündungsstoffe und -zellen beteiligt sind. Unter anderem werden Enzyme gebildet, die die Bakterien zerstören sollen, jedoch auch zu einer Zerstörung von Eigengewebes führen. Das führt letztendlich zum Verlust von Bindegewebe und Knochen. Das Ergebnis der Reaktion auf die Bakterien sind Zahnfleischbluten, Taschenbildung, zurückgehendes Zahnfleisch und schließlich Lockerung und Verlust der Zähne.

Jenseits der fünfunddreißig verlieren mehr Menschen Zähne durch Parodontitis als durch Karies. Weitgehend unbekannt ist, dass krankes Zahnfleisch auch andere Organe schwer belasten kann. Parodontitis ist eine sehr häufige Erkrankung, bei der im Laufe der Jahre durch mangelnde Mundhygiene das Zahnfleisch bzw. der Zahnhalteapparat geschädigt wird. Besonders ohne eine Parodontitisbehandlung besteht die Gefahr des Verlustes von Zähnen.

In den meisten Fällen handelt es sich um ein in chronischer Weise verlaufendes Geschehen. Dieses tritt vorwiegend bei Erwachsenen auf, ist nur selten schmerzhaft und führt von den Betroffenen zumindest meist unbemerkt, erst nach Jahren zu Zahnlockerungen. Der Zahnfleischsaum bietet daher für Bakterien einen relativen Schutz vor der selbstreinigenden Mundhöhle durch Zunge und Speichel. Beim Gesunden garantiert das so genannte Saumepithel durch seine Anhaftung am Schmelz eine kontinuierliche Oberfläche zwischen Zahnfleisch und Zahn. Wird die Plaque in diesen Nischen nicht sorgfältig entfernt, greifen die Ausscheidungsprodukte der Mikroorganismen (Exotoxine) das Saumepithel an und einige Bakterien sind sogar in der Lage das Epithel zu durchwandern. Der Körper reagiert auf solche Angriffe mit der Einwanderung von Abwehrzellen aus dem Blut. Nach und nach werden so die Eindringlinge zerstört und phagozytiert. Dabei werden verschiedene Endotoxine freigesetzt. Sowohl die Exotoxine als auch die Endotoxine und einige Zerfallsprodukte der Körperabwehrzellen stellen einen Reiz dar. Um das umliegende Gewebe von diesen Reizen zu schützen und ein Vordringen der Entzündung in die Kiefer vorzubeugen, aktiviert der Körper unter anderem auch Osteoklasten. Deren Aufgabe besteht in zielgerichteten Ab- und Umbau von Knochengewebe.

Bei einer guten Körperabwehr können die Mikroorganismen lange davon abgehalten werden, in die Kiefer vorzudringen. Die Kräfteverhältnisse in diesem Kampf sind jedoch sehr labil. Eine Verschlechterung der Körperabwehr, eine starke Vermehrung von Bakterien, oder eine Veränderung der Aggressivität der Mikroorganismen führt dann zu einem weiteren Fortschreiten des Entzündungsgeschehenes in die Tiefe. So kommt es im Verlauf zu einem stetigen Knochenverlust, der nur durch eine vollständige Entfernung der Reize gestoppt werden kann. Auf Röntgenbildern erscheint der Knochenverlust vorwiegend horizontal, da die Osteoklasten in Ruhephasen der Entzündung das zerklüftete Knochengewebe ausformen und so an die neuen Gegebenheiten anpassen. Aufgrund des langsamen und langen Krankheitsverlaufes wird diese Form der Entzündung als chronische Parodontitis bezeichnet. Davon wird die aggressive Parodontitis unterschieden, welche rasch zu umfangreichem Knochenverlust führt und manchmal auch schon in Kindesalter auftritt.

Von den etwa 500 verschiedenen Bakterienspezies, die in der Mündhöhle vorkommen können, sind nur wenige parodontalpathogen. Diese werden auch als Hauptleitkeime bezeichnet und bilden so genannte Cluster, welche in ihrer Vergesellschaftung spezifisch sind. Sie sind obligat oder fakultativ anaerobe, gramnegative, schwarzpigmentierte Bakterienarten, wie der so genannte rote Komplex (Porphyromonas gingivalis, Treponema denticola und Tannerella forsythenisis sowie Actinobacillus actinomycetemcomitans Subtyp B).

Die Therapie besteht heute darin, den Entzündungszustand des Zahnfleisches und des Zahnhalteapparates zu beseitigen und Plaque und Zahnstein, sowie entzündungsfördernde Faktoren zu beseitigen. Die Behandlung gliedert sich in verschiedene Phasen, in denen unterschiedliche Maßnahmen ergriffen werden können. Die erste Phase stellt eine umfassende Diagnostik dar, mit welcher Art, Schwere und Verlauf der Erkrankung bestimmt werden können. Neben Röntgenaufnahmen folgt noch eine klinische Beurteilung des Gesamtzustandes des Gebisses. Inzwischen werden in vielen Fällen ergänzend mikrobiologische (Nachweis bestimmter parodontalpathogener Bakterien) Tests durchgeführt. Ist die Parodontitis so stark ausgeprägt, dass Antibiotika verabreicht werden müssen, ist es von Vorteil vorher eine Keimbestimmung durchzuführen, damit zielgerichteter behandelt werden kann.

Unbehandelt führt die Parodontitis fast immer zu Zahnverlust und daraus folgend zu ästhetischen und funktionellen Beeinträchtigungen. Außerdem ist Parodontitis ein Risikofaktor für allgemein medizinische Erkrankungen. So gilt ein Zusammenhang zwischen parodontalen Erkrankungen und dem erhöhten Risiko für das Auftreten von Herzinfarkten und Erkrankungen des rheumatischen Formenkreises als wissenschaftlich gesichert. In neueren Untersuchungen konnte außerdem gezeigt werden, dass eine unbehandelte Parodontitis das Risiko von Frühgeburten um das siebenfache steigert und auch ein niedriges Geburtsgewicht ursächlich mit der Parodontitis zusammenhängen kann.

Aus der AT 411 174 B ist ein Verfahren und ein Kit zur Analyse von Nukleinsäuren, welche zur Identifikation von mit Parodontitis assoziierten Bakterien verwendet werden, bekannt. Hier wird auf die Oberfläche eines Trägers, auf welche zumindest ein vordefinierter Analysebereich und vordefinierter Kontrollbereich angeordnet sind, ein Oligonukleotid mit einer Sequenz aufgebracht, welches zur Zielnukleinsäuresequenz komplementär ist. Es werden Oligonukleotid für die Identifizierung von diverser mit Parodontitis assoziierter Bakterien angeführt, die bevorzugt aus 10 bis 120 Nukleotiden bestehen, aufgebracht. Als Träger wird ein Biochip beschrieben, auf welchem die spezifischen Oligonukleotide gespottet werden. Bevor die biologische Probe mit dem Biochip in Kontakt kommt, wird mittels spezifischen Primersequenzen die in der biologischen Probe vorkommende Bakterien DNA amplifiziert.

Die DE 699 24 741 T2 beschreibt eine Zusammensetzung zur Herstellung eines Arzneimittels für die Verhütung und Behandlung der Parodontitis. Hierbei wird die Parodontitis durch die Verwendung eines, einen antimikrobiellen Wirkstoff enthaltenden, Lacks wirksam bekämpft. Es wird eine antimikrobielle Zusammensetzung verwendet, die eine physiologisch verträgliche Klarlackgrundlage und darin gelöst einen antimikrobiellen Wirkstoff umfasst.

Auch aus der DE 101 54 290 A1 ist ein Verfahren zum Nachweis von Parodontitis und Karies assoziierter Bakterien bekannt. Die Erfindung betrifft insbesondere Hybridisierungsverfahren und Amplifikationsverfahren sowie gekoppelte Amplifikations-/ Hybridisierungsverfahren mit sequenzspezifischen Sonden bzw. Primern. Für die Amplifikation wird bevorzugt die Polymerasekettenreaktion eingesetzt. In der einfachsten Form der Detektion der nachzuweisenden Nukleinsäure wird das Amplifikat z. B. durch Verdauung mit einem Restriktionsenzym spezifisch geschnitten und die entstandenen Fragmente auf einem Agarosegel analysiert. Weit verbreitet sind auch Hybridisierungssysteme, wobei die Hybridisierung üblicherweise so stattfindet, dass entweder die Zusammensetzung, die das Amplifikationsprodukt oder einen Teil davon enthält oder die Sonde auf einer festen Phase immobilisiert wird und mit dem jeweils anderen Hybridisierungspartner in Kontakt gebracht wird. Als feste Phase sind verschiedene Materialien vorstellbar, wie beispielsweise eine Mikrotiterplatte. Die Zielsequenz kann dabei auch zuvor in Lösung mit einer Fangsonde hybridisiert werden und danach wird die Fangsonde an einer festen Phase gebunden. In der Regel ist wenigstens eine Sonde oder wenigstens ein Primer bei der Amplifikation oder nachzuweisenden Nukleinsäure markiert.

Die WO 2007/056680 A beschreibt ein Verfahren und Arrays zur Detektion der menschlichen Mikroflora, unter anderem auch Oligonukleotidsequenzen zur Detektion von Parodontitis.

Ferner beschreibt die WO 89/06704 A Oligonukleotide, die mit Parodontitis verursachenden Keimen assoziiert sind, insbesondere die Oligonukleotidsequenzen Bf-3B. Bg-3B und Bi-5B. Es werden auch Teststreifen bestehend aus einer festen Phase, an der mindestens eine unmarkierte Oligonukleotidsonde gebunden ist, welche spezifisch an die 16S rRNA oder 23 S rRNA des nachzuweisenden Bakteriums bindet. Darüberhinaus wird ein Verfahren zum Nachweis von mit peridontalen Krankheiten assoziierten Bakterien beschrieben, wobei eine aus dem Mund eines Patienten gewonnene Probe lysiert wird und die freigesetzte rRNA mit speziesspezifischen Sonden hybridisiert wird. Der Nachweis von Hybridisierungskomplexen zeigt die Anwesenheit eines entsprechenden Bakteriums in der Probe an.

Aufgabe der vorliegenden Erfindung ist es daher, ein Verfahren und eine Vorrichtung zum Nachweis und/oder Bestimmung von Parodontitis assoziierten Bakterien zu Verfügung zu stellen, wobei das Ergebnis rasch vorliegt und die Vorrichtung einfach herzustellen und zu handhaben ist.

Die erfindungsgemäße Aufgabe wird jeweils eigenständig gelöst durch ein Verfahren zum Nachweis und/oder zur Bestimmung von mit Parodontitis assoziierten Bakterien aus einer biologischen Probe mit zumindest einem der Oligonukleotide SEQ ID No. 4 oder SEQ ID No. 5; durch eine mikrofluidische Einrichtung umfassend einen Träger aus zumindest einem Bodenteil mit einer Oberfläche und mindestens ein an die Trägeroberfläche gebundenes Oligonukleotid oder Nukleinsäuremolekül, dadurch gekennzeichnet, dass zumindest eines der Oligonukleotide SEQ ID No. 4 oder SEQ ID No. 5 gebunden ist sowie optional mindestens ein weiteres Oligonukleotid, welches ausgewählt ist aus der Gruppe enthaltend: i) Oligonukleotid SEQ ID No. 1, 2, 3, 6, 7 oder 8, ii) Oligonukleotid, das ein gegenüber einem der Oligonukleotide SEQ ID No. 1, 2, 3, 6, 7 oder 8 mutierte Nukleotidsequenz aufweist, insbesondere eine Addition oder Deletion von 1 bis 10 Nukleotiden oder eine Substitution von 1 bis 3 Nukleotiden in einer der Nukleotidsequenzen, iii) Oligonukleotid, das eine Nukleotidsequenz aufweist, die zumindest bereichsweise zu der Nukleotidsequenz SEQ ID No. 1, 2, 3, 6, 7 oder 8 komplementär ist; sowie durch einen kit umfassend a) zumindest einen Träger, vorzugsweise eine mikrofluidische Vorrichtung, mit zumindest einem immobilisierten Oligonukleotid oder einer Nukleinsäuresequenz der SEQ ID No. 4 oder SEQ ID No. 5 sowie optional mindestens einem weiteren Oligonukleotid, welches ausgewählt ist aus der Gruppe enthaltend: i) Oligonukleotid SEQ ID No. 1, 2, 3, 6, 7 oder 8, ii) Oligonukleotid, das ein gegenüber einem der Oligonukleotide SEQ ID No. 1, 2, 3, 6, 7 oder 8 mutierte Nukleotidsequenz aufweist, insbesondere eine Addition oder Deletion von 1 bis 10 Nukleotiden oder eine Substitution von 1 bis 3 Nukleotiden in einer der Nukleotidsequenzen, iii) Oligonukleotid, das eine Nukleotidsequenz aufweist, die zumindest bereichsweise zu der Nukleotidsequenz SEQ ID No. 1, 2, 3, 6, 7 oder 8 komplementär ist; und b) zumindest einen Behälter mit einer Universallösung, welche zumindest ein chaotropes Reagenz sowie zumindest ein Oligonukleotid umfasst.

Vorteilhaft dabei erweist sich, dass durch die speziesspezifischen Sequenzen eine exakte Identifikation der Bakterien möglich ist und somit eine eindeutige Zuordnung zu einem pathologischen Muster erfolgen kann.

In einer Weiterbildung umfasst das Verfahren folgende Schritte: a) Transfer der biologischen Probe in eine Universallösung in einen Behälter, wobei zumindest ein Sandwicholigonukleotid, vorzugsweise Oligonukleotid SEQ ID No. 6 enthalten ist, b) Denaturieren der Probe, insbesondere durch Erhitzen des Inhalts des Behälters, c) Transfer zumindest eines Teils der Lösung in bzw. auf einen Träger mit zumindest einem immobilisierten Oligonukleotid SEQ ID No.4 oder SEQ ID No. 5 und gegebenenfalls SEQ ID No. 1, 2, 3, 7 und/oder 8, d) Hybridisierung und gegebenenfalls Inkubation, und e) Detektion. Dabei erweist sich von Vorteil, dass die biologische Probe durch das Oligonukleotid SEQ ID No. 6, welches an eine hochkonservierte Region bindet, markiert wird und durch die speziesspezifischen Sonden, die auf dem Träger immobilisiert sind, gebunden und detektiert und bestimmten Bakterien zugeordnet werden kann. Durch Kontrollsequenzen, wie Oligonukleotid SEQ ID No. 7 und SEQ ID No. 8, kann eine Kontrolle der Hybridisierung bzw. generell des Verfahrens erfolgen.

Durch das Aufbringen zumindest einer weiteren Reaktionslösung, wie Markierungslösung, Enzymlösung, Waschlösung, Farbreaktionslösung, in bzw. auf den Träger wird das Verfahren verbessert, indem beispielsweise bei der Detektion störende Signale durch die Waschlösung vermieden werden können, bzw. durch eine korrespondierende Enzym- und Farbreaktionslösung die Nachweisgrenze herabgesetzt werden kann.

Die Analyse einer in der biologischen Probe enthaltenen Nukleinsäure erfolgt durch Hybridisierung mit mindestens einer Sonde, wobei die Sonde ausgewählt ist aus einer Gruppe enthaltend i) Oligonukleotid SEQ ID No. 4 oder SEQ ID No. 5 Vorteilhaft dabei erweist sich, dass der Nachweis und/oder die Bestimmung jener mit Parodontitis assoziierten Bakterien sehr rasch, vorzugsweise in weniger als 15 min erreicht werden kann. Von Vorteil ist zudem, dass die Nachweismethode sehr sensitiv ist und Bakterien bereits in einer Anzahl von weniger als 10⁵ Bakterien nachgewiesen werden können. Neben der raschen Durchführbarkeit des Verfahrens ist dieses auch sehr preiswert und erlaubt eine einfache Handhabung, wobei keine spezielle Laborausrüstung erforderlich ist. Das erfindungsgemäße Verfahren kann somit auch in einer Zahnarztordination durchgeführt werden und das Ergebnis dem Probanden noch beim selben Termin sofort mitgeteilt werden. Der Nachweis des Analyten ist durch visuelle Auswertung möglich.

Als Träger kann ein Kapillarsystem, wie Teststreifen oder mikrofluidische Vorrichtung, verwendet werden, wodurch eine einfache Vorrichtung zum Nachweis von mit Parodontitis assoziierten Keimen verwendet werden kann, weil keine zusätzlichen Hilfsmittel, wie beispielsweise ein Netzanschlussgerät zur Einstellung und/oder Regelung von Strom und/oder Spannung oder dergleichen, zum Transport der Probe erforderlich sind.

Als Sonde kann ein Oligonukleotid oder Nukleinsäuremolekül, welches ein DNA-, RNA-, PNA-, LNA-Molekül oder eine Mischform ist, verwendet werden, weil LNA-Oligonukleotide sowie PNA-Moleküle dem Watson-Crick-Model entsprechen und an komplementäre Oligonukleotide hybridisieren. LNA/DNA- oder LNA/RNA-Duplexmoleküle zeigen gegenüber ähnlichen Duplexmolekülen, die ausschließlich aus DNA oder RNA gebildet werden, erhöhte thermische Stabilität.

Vorteilhaft erweist sich, dass die biologische Probe eine subgingivale Plaqueprobe, insbesondere aus dem Parodontium, darstellt, weil diese somit durch nicht-invasive Methoden gewonnen werden kann und somit von jedermann jederzeit zur Verfügung gestellt werden kann.

Weiters erweist sich von Vorteil, dass die der biologischen Probe zugewandte Oberfläche der mikrofluidischen Vorrichtung zumindest bereichsweise hydrophilisiert, insbesondere mit Plasmapolymerisation modifiziert, ist, wodurch ein kapillarer Flüssigkeitstransport der biologischen Probe auf einer mikrofluidischen Vorrichtung ermöglicht, bzw. zumindest stark erleichtert wird. Hierbei erweist es sich als besonders vorteilhaft, dass der kapillare Flüssigkeitstransport kontinuierlich verläuft und es zu keinem Abreißen des Flüssigkeitsstroms im Kanal der mikrofluidischen Vorrichtung kommt.

Es wird zumindest ein steriles Probenentnahmeelement, vorzugsweise eine Papierspitze, Kürette, Spatel oder dergleichen an einen Taschenfundus vorgeschoben und ggf. dort verweilen, wodurch mittels einen einfachen jedoch weder spitzen noch scharfen Gegenstand eine biologische Probe gewonnen werden kann, welche keine Verletzungen verursacht bzw. das womöglich stark mitgenomme bzw. angegriffene Gewebe zusätzlich verletzt.

In der Universallösung ist vorzugsweise ein chaotropes Reagens, wie Guanidiniumsalze, wie Guanidiniumisothiocyanat, Formamid, Harnstoff, Perchlorat, Thiocyanat, Trichloroacetat, Nitrat, Iodid, etc. in einer Konzentration von 0,1 M bis 10 M enthalten, wodurch stringente Bedingungen für eine Hybridisierung bei Raumtemperatur geschaffen werden und andererseits die Zelllysis erfolgen kann. Durch die Verwendung von Guanidiniumthiocyanat bzw. Harnstoff wird die Anwendung teratogener Substanzen obsolet und somit Schäden bei den die Lösung manipulierenden Personen verhindert.

Weiters erweist sich von Vorteil, dass in der Universallösung eine Markierung, insbesondere durch das markierte Oligonukleotid SEQ ID No. 6, für die biologische Probe enthalten ist, wodurch bereits in einem Arbeitsschritt sowohl die Lyse der biologischen Probe als auch die Markierung der Nukleinsäure erfolgen kann.

Viele der Schritte, insbesondere die Hybridisierung können bei Raumtemperatur durchgeführt werden, wodurch die Möglichkeit als Side-Chairtest bestätigt wird und nur für die Durchführung eines Arbeitsschrittes (Lyse) eine Heizquelle erforderlich ist.

Es ist vorgesehen, den Behälter auf eine Temperatur von mind. 50°C zu erhitzen, wodurch einerseits der Zellaufschluss der biologischen Probe unterstützt wird und andererseits Nukleinsäuremoleküle denaturiert werden.

Vorzugsweise erfolgt der Transfer der noch erhitzten biologischen Probe auf den Träger, vorzugsweise die mikrofluidische Vorrichtung, mittels einer Pipette oder Kapillare, wodurch gewährleistet wird, dass die biologische Probe noch im denaturierten Zustand vorliegt.

Es kann eine Inkubation für eine Dauer von 1 min bis 10 min erfolgen, wodurch ein ausreichend langer Zeitraum für das Finden zueinander komplementärer Nukleinsäuremoleküle zur Verfügung steht.

Weiters ist vorgesehen, dass die Enzym-, Wasch-, und Farbreaktionslösung jeweils mit einer Tropfflasche aufgebracht werden und die mikrofluidische Vorrichtung mit der jeweiligen Lösung vorzugsweise für eine Dauer von 5 sec bis 10 min inkubiert wird, wodurch die für den visuellen Nachweis des Analyten erforderlichen Reaktionen ablaufen können.

In einer bevorzugten Ausführungsform erfolgt die Detektion kolorimetrisch, wobei zu handelsüblichen Tetramethylbenzidin-Rekationsmischungen Tetramethylbenzidin, vorzugsweise in Polyvinylpyrrolidon gelöst, der Farbreaktion zugesetzt wird, wodurch durch die Bildung von Komplementärfarben das visuelle Signal verstärkt wird.

Weiters ist vorgesehen, dass das Verfahren zur Diagnose und/oder Früherkennung von Erkrankungen, Erkrankungsvorstufen, Erkrankungsrisiken und/oder krankhaften Veränderungen, die von Parodontitis verursachenden Bakterien ausgehen, eingesetzt wird, wodurch bereits frühzeitig das Risiko für eine Parodontitiserkrankung erkannt werden kann.

Durch die Auswahl der spezifischen Sequenzen für die Bakterienstämme Tannerella forsythensis, Porphyromonas gingivalis, Prevotella intermedia, Treponema denticola und Hemophilus actinomycetemcomitans wird sichergestellt, dass jene Bakterien im Mundraum bzw. in der Zahntasche detektiert werden die tatsächlich mit Parodontitis assoziiert werden.

Durch die Verwendung der Universalsonde SEQ ID No. 8 werden möglichst alle im Mundraum vorkommenden Bakterien detektiert. Somit wird sichergestellt, dass sämtliche Bakterien, welche sich in der Zahntasche befinden, allerdings keine komplementäre Sequenz zu den Oligonukleotiden SEQ ID Nr. 1 bis SEQ ID Nr. 7 aufweisen, nachgewiesen werden können.

Weiters ist vorgesehen, dass an der Trägeroberfläche der mikrofluidischen Vorrichtung eine Kontrolle wie Positiv-, Negativ-, Orientierungs-, Hybridisierungs-, und/oder Farbreaktionskontrolle oder dgl. angeordnet ist, wodurch nachgewiesen werden kann, dass der Test an sich funktionierte, aber da bspw. bei den bakterienspezifischen Sonden keine Bindung erfolgte, keine Parodontitis assoziierten Bakterien in der biologischen Probe vorkommen.

Vorteilhaft erweist sich zudem, dass im Kit zumindest eine weitere Reaktionslösung, wie Wasch-, Enzym-, Farbreaktionslösung, und/oder dgl. enthalten ist, wodurch bereits alle Reagenzien, die für die Durchführung der Analyse bzw. zur Bestimmung und zum Nachweis der Parodontitis assoziierter Bakterien erforderlich sind, im Kit enthalten sind und keine zusätzlichen Reagenzien separat gekauft werden müssen.

Für den Anwender ist es lediglich erforderlich, eine Beheizungsquelle für die biologische Probe zur Verfügung zu stellen, um eine Temperatur von mind. 50°C für den Zellaufschluss bzw. für die Denaturierung der Nukleinsäure zu erzielen. Das erfindungsgemäße Verfahren ermöglicht eine Detektion von mit Parodontitis assoziierten Bakterien ohne vorher eine Amplifikation durchführen zu müssen, wie es aus dem Stand der Technik bekannt ist.

Einführend sei festgehalten, dass in den unterschiedlich beschriebenen Ausführungsformen gleiche Teile mit gleichen Bezeichnungen versehen werden, wobei die in der gesamten Beschreibung enthaltenen Offenbarungen sinngemäß auf gleiche Teile mit gleichen Bezeichnungen übertragen werden können. Auch sind die in der Beschreibung gewählten Lageangaben, wie z.B. oben, unten, seitlich usw. auf die unmittelbar beschriebene sowie dargestellte Figur bezogen und sind diese bei einer Lageänderung sinngemäß auf die neue Lage zu übertragen. Weiters können auch Einzelmerkmale oder Merkmalskombinationen aus den gezeigten und beschriebenen, unterschiedlichen Ausführungsbeispielen für sich eigenständige, erfinderische oder erfindungsgemäße Lösungen darstellen.

Sämtliche Angaben zu Wertebereichen in gegenständlicher Beschreibung sind so zu verstehen, dass diese beliebige und alle Teilbereiche daraus mit umfassen, z.B. ist die Angabe 1 bis 10 so zu verstehen, dass sämtliche Teilbereiche, ausgehend von der unteren Grenze 1 und der oberen Grenze 10 mit umfasst sind, d.h. sämtliche Teilbereich beginnen mit einer unteren Grenze von 1 oder größer und enden bei einer oberen Grenze von 10 oder weniger, z.B. 2 bis 9 oder 4 bis 7 oder 5 bis 6.

Es wird eine subgingivale Plaqueprobe von der jeweils tiefsten Stelle der parodontalen Tasche gegebenenfalls mit Blutung auf Sondierung und/oder Suppuration, vorzugsweise in jedem Quadranten, entnommen. Akut blutende und/oder stark eiternde Stellen werden vermieden. Die biologische Probe wird vorzugsweise vom Zahnarzt oder dessen Assistentin mit einer Papierspitze vom Taschenfundus gewonnen. Es können auch andere Mittel zur Probenentnahme, wie ein Wattestäbchen, Tupfer, Kürette, etc. verwendet werden.

Es können auch mehrere biologische Proben gewonnen werden und gemeinsam dem erfindungsgemäßen Verfahren zugeführt werden. Von Vorteil erweist sich jedoch max. 2 Papierspitzen pro Testdurchlauf zu verwenden, wodurch max. 2 verschiedene Entnahmestellen zu einer Mischprobe zusammengeführt werden und auf den Träger aufgebracht werden. Sollen weitere Stellen getestet werden, so werden vorzugsweise die weiteren Papierspitzen in weitere Proberöhrchen zugeführt und ein neuer Träger verwendet. Soll das erfindungsgemäße Verfahren zu Screeningzwecken eingesetzt werden, erfolgt die Probennahme vorzugsweise mit einer Kürette.

Nach Trockenlegung und Entfernung der supragingivalen Plaques wird der Entnahmeort mit Watterollen isoliert, um subgingivalen Plaques zu entnehmen. Danach werden die sterilen Papierspitzen mit einer Pinzette bis zum Taschenfundus vorgeschoben und dort für ca. 15 Sekunden (sec) belassen. Die Papierspitzen werden mit der Pinzette wiederum entnommen und dem Proberöhrchen zugeführt. In dem Proberöhrchen befindet sich die Universallösung entweder vorgelegt oder diese wird zugefügt. Die mit der biologischen Probe in Kontakt gebrachte Papierspitze muss in die Lösung im Proberöhrchen eintauchen. Es wird vorzugsweise ein 500 µl Proberöhrchen verwendet, in welchem ein Aliquot der Universallösung, vorzugsweise zwischen 10 µl und 300 µl, vorgelegt sind. Das Proberöhrchen wird verschlossen und gemischt, wie z.B. kräftig geschüttelt und/oder gevortext, gerührt, resuspendiert, etc. Zuletzt wird die Flüssigkeit am Boden des Probenröhrchens gesammelt und die Flüssigkeit samt der biologischen Probe im Proberöhrchen erhitzt. Das Proberöhrchen wird vorzugsweise auf eine Temperatur ausgewählt aus einem Bereich mit einer unteren Grenze von 72°C und einer oberen Grenze von 100°C, vorzugsweise 90°C, insbesondere 95°C, erhitzt. Um eine vollständige Lyse und Denaturierung der biologischen Probe zu erzielen wird vorzugsweise für einen Zeitraum von mind. 10 sec bis max. 5 min erhitzt.

Nach der Erhitzung wird die noch heiße Flüssigkeit entnommen, und auf den Träger, vorzugsweise die Probenaufgabeöffnung der mikrofluidischen Vorrichtung aufgebracht.

Die mikrofluidische Vorrichtung umfasst zumindest einen Bodenteil und/oder Deckelteil. Auf bzw. im Bodenteil und/oder Deckelteil ist ein Kanal angeordnet. Der Kanal verbindet die Probenaufgabeöffnung mit einer am der Probenaufgabeöfnung gegenüberliegenden Ende angeordneten Verbreiterung.

Die Entnahme der Probe erfolgt vorzugsweise mittels einer Pipette oder Kapillare. Es wird der biologischen Probe bzw. der Flüssigkeit die Möglichkeit gegeben, über Kapillarkraft in den Kanal der mikrofluidischen Vorrichtung gesogen zu werden. Hierzu erfolgt eine Inkubation von mind. 10 sec, vorzugsweise mind. 1 min.

Nach dieser Inkubationszeit wird eine Enzymlösung in die Probenaufgabeöffnung der mikrofluidischen Vorrichtung aufgebracht. Abermals nach einer Inkubation von mind. 10 sec, wobei in dieser Zeit die Enzymlösung ebenfalls in den Kanal gesogen wird, wird bspw. eine Waschlösung in die Probenaufgabeöffnung der mikrofluidischen Vorrichtung aufgebracht und wiederum inkubiert, um ein Einsaugen der Flüssigkeit in den Kanal zu ermöglichen.

Zuletzt wird die Farbreaktionslösung auf die Probenaufgabeöffnung der mikrofluidischen Vorrichtung aufgebracht und es kann die Farbreaktion bis zur optimalen Sichtbarkeit des Signals abgewartet werden.

Das Aufbringen der Enzymlösung, der Waschlösung und der Farbreaktionslösung kann mittels einer Pipette bzw. einer Tropfflasche erfolgen. Je nach Dauer der einzelnen Inkubationsschritte ist das erfindungsgemäße Verfahren bereits nach 1 min bis 2 min abgeschlossen. Allerdings können die Inkubationszeiten auch verlängert werden, dass das Ergebnis der Analyse erst nach 10 min oder mehr Minuten vorliegt. Das Analyseergebnis kann visuell abgelesen werden, bzw. mit in einem Desktopscanner eingescannt bzw. ausgewertet werden.

Die Universallösung umfasst zumindest ein chaotropes Reagens sowie zumindest ein Oligonukleotid. Die Universallösung kann beispielsweise Guanidiniumthiocyanat, Tris- HCl, DNA und ein markiertes Oligonukleotid enthalten. Ähnliche Lösungen sind beispielsweise aus der US 5,334,501 bekannt.

Die Enzymlösung umfasst einen Peroxidasekonjungatstabilisierer und Streptavidin Peroxidase.

Um Waschlösung herzustellen werden Natriumchlorid, Natriumcitrat und Proclin 200 abgewogen und mit Wasser versehen.

Für die Herstellung der Farbreaktionslösung werden TMB und TMB/PVP-Lösung und Proclin 200 vermischt bzw. kann auch eine kommerziell erhältliche niederschlagsbildende TMB-Lösungen verwendet werden.

Als Oligonukleotidsequenzen werden vorzugsweise die nachfolgend angeführten Sequenzen verwendet, wobei mindestens 15 der angegebenen aufeinander folgenden Nukleotide des jeweiligen Oligonukeotids, deren komplementäreund/oder mutierte Sequenzen oder dgl. verwendet werden.

Die Sandwich-Sonde dient zum Labeln der 16 S rRNA der biologischen Probe. Sie ist mit Biotin am 5' Ende gelabelt. Die Sandwich-Sonde weist die Sequenz 5'-CATCG AATTA AACCA CATGY TCCWC CGCTT GT-3' (SEQ ID No. 6) auf. Die Sandwichsonde bindet an die biologische Probe und weil die Sandwichsonde selbst markiert ist, wird auch die biologische Probe markiert. Vorzugsweise wird die biologische Probe mittels des Oligonukleotids SEQ ID No. 6 in der Universallösung markiert. Das Oligonukleotid SEQ ID No. 6 trägt selbst die Markierung/Label. Diese Markierung kann entweder selbst (direkt) detektiert werden, oder durch weitere Bindungsschritte (indirekt) detektiert werden.

Als Kontrollsondensequenz für die Hybridisierungskontrolle wird ein Oligonukleotid mit der Sequenz 5'-ACAAG CGGWG GARCA TGTGG TTTAA TTCGA TG-3' (SEQ ID No. 7) verwendet. SEQ ID No. 7 ist teilweise komplementär zu SEQ ID No. 6 und dient daher als Kontrolle und ist vorzugsweise auf dem Träger immobilisiert.

Als Kontrolle für das Enzymkonjugat wird ein biotinyliertes Oligonukleotid mit beliebiger Sequenz oder ein mit der Oberfläche reagierendes Biotin-Derivat verwendet.

Die Oligonukleotide SEQ ID No. 1 bis 5 sind bakterienspezifische Sonden. Für den Nachweis des Bakterienstammes Tannerella forsythensis wird ein Oligonukleotid mit der Sequenz 5'-AAGAA AGCTC TCACT CTCCG TCGTC TA-3' (SEQ ID No. 1) verwendet.

Als spezifische Sonde für Porphyromona gingivalis wird ein Oligonukleotid mit der Sequenz 5'-CGCTG TGGAA GCTTG ACGGT ATATC GCAAA CTC-3' (SEQ ID No. 2) verwendet.

Für den Nachweis des Bakterienstammes Prevotella intermedia wird ein Oligonukleotid mit der Sequenz 5'-AGTCA ACATC TCTGT ATCCT GCGTC TGCA-3' (SEQ ID No. 3) verwendet.

Als spezifisches Oligonukleotid für den Bakterienstamm Treponema denticola wird ein Oligonukleotid mit der Sequenz 5'-AAGA GCCGT ATTGC TACGC TGCCA TATCT CTA-3' (SEQ ID No. 4) verwendet.

Als Bakterienstamm spezifische Sonde für den Nachweis von Hemophilus actinomycetemcomitans wird das Oligonukleotid mit der Sequenz 5'-GTCTC AAGC TCCCT AAGGC TCAAA CCCAT C-3' (SEQ ID No. 5) eingesetzt.

Die Universalsonde, die möglichst alle im Mundraum vorkommenden Bakterienstämme nachweisen soll und auch als Kontrollsonde dient, weist die Sequenz 5'-CCCGT CAATT CMTTT GAGTT TYAMC STTGC-3' (SEQ ID No. 8) auf. Die Universalsonde ist vorzugsweise auch auf dem Träger immobiliert.

Zur Markierung der biologischen Probe wird ein Label-Reagenz verwendet, das vorzugsweise aus einem Label, einer reaktiven Gruppe und aus einem Linker zwischen den beiden Gruppen besteht. Die reaktiven Gruppen sind Psoralene, Arylazide, reaktive Gruppen aus den Mustardverbindungen (Label-IT), Cis-Platin-Komplexe (Universal Label System), etc. oder beispielweise ein Sandwich-Oligonukleotid verwendet, welches an einer anderen als der spezifischen Sondenposition auf den Target hybridisiert.

Neben den bereits beschriebenen visuellen, insbesondere kolorimetrischen Detektionsmethoden mit Tetramethylbenzidin (TMB) können auch noch andere Reagenzien wie Diaminobenzidine (DAB), Diaminobenzidine mit nachfolgender Silberspiegelreaktion, Silver enhancement (amplification) auf Gold Nanopartikeln, Standardsubstrate für Horseradish-Peroxidase und alkalischer Phosphatase, etc. verwendet werden. Eine Detektionsmethode auf Lumineszensbasis wird beispielsweise bei der Lichterzeugung durch Abbau von Luminol durch Horseradish-Peroxidase erreicht. Die Detektion dieses Lichtes erfolgt über Fotopapier oder Fotodioden.

Um die kolorimetrische Detektionsmethode, insbesondere die Tetramethylbenzidinmethode, zu verbessern wird folgendes vorgeschlagen: Die Oxidation von TMB durch HRP und Wasserstoffperoxid läuft über zwei Stufen ab. Zuerst bildet sich ein Komplex aus einem oxidierten und einem nicht oxidierten TMB-Molekül, der intensiv blau gefärbt ist. Bei fortlaufender Reaktion wird TMB zu einem gelben Produkt oxidiert. Bei kommerziell erhältlichen TMB-Reaktionsmischungen ist Wasserstoffperoxid in einem molaren Überschuss vorhanden. Die Reaktion läuft daher beginnend bei einem blauen Zwischenprodukt bis zum gelben Endprodukt. Die Farbe ändert sich also von blau über grün zu gelb. Durch Zufügen von TMB zu dieser Reaktionsmischung kann die Reaktion beim blauen Zwischenprodukt gestoppt werden. Dazu wird in 1 % Polyvinylpyrrolidon gelöstes TMB zugesetzt. Die Reaktion stoppt nun beim blauen, für das Auge viel besser sichtbaren Zwischenprodukt.

Wie bereits erwähnt, muss die Oberfläche des Trägers hydrophil sein, damit die Flüssigkeit in den Kanal gesogen werden kann und zugleich die Nukleinsäurebindung verbessert wird. Von Vorteil erweist sich auch, dass die verwendete Hydrophilisierungsmethode für die Modifikation von Kunststoffoberflächen verwendbar ist. Diese notwendige Hydrophilisierung wird sowohl durch die Plasmapolymerisation als auch bspw. durch aus dem Stand der Technik bekannte Oberflächenmodifikationen, wie z.B. der Fa. PolyAn, erzielt. Bei einer unfunktionalisierten Oberfläche bleibt die Flüssigkeit in der Probenaufgabeöffnung zurück und es kommt kein kapillarer Flüssigkeitstransport zustande.

Zudem ergibt sich durch die Hydrophilisierung der Oberfläche des Trägers der mikrofluidischen Vorrichtung, insbesondere die Plasmapolymeristion, eine Gelbfärbung der sonst weißen Oberfläche des Trägers der mikrofluidischen Vorrichtung. Diese Gelbfärbung verstärkt das Signal, da die Farbe des Niederschlags, wie bereits vorab erwähnt, blau ist. Mit der Komplementärfarbe gelb ergibt sich somit gegebenenfalls eine dunklere Färbung des Niederschlags.

In Vergleichsversuchen zwischen der Plasmapolymerisation und der Behandlung der Trägeroberfläche mit Polyan zur Hydrophilisierung konnte festgestellt werden, dass mit der Plasmapolymerisation mindestens genau so gute Immobilisierungseigenschaften sowie Hybridisierungseigenschaften der immobilisierten DNA-Sonden erzielt werden konnten. Zudem konnte festgestellt werden, dass durch die Plasmapolymerisation eine niedrigere unspezifische Adsorption von Nukleinsäuren als auch Proteinen auf der Oberfläche der mikrofluidischen Vorrichtung erfolgt. Die Durchführung einer Oberflächenmodifikation mittels Plasmapolymerisation ist in der österreichischen Patentanmeldung A0 1619/2007 der Anmelderin beschrieben und deren Inhalt somit zum Umfang dieser Anmeldung zählt.

### Anwendungsbeispiel:

Die vom Zahnarzt mit einer Papierspitze entnommene Probe wird in ein 500 µl Proberöhrchen gegeben und mit 60 µl Universallösung versetzt. Das Proberöhrchen wird verschlossen und für 20 s kräftig geschüttelt. Durch eine Schüttelbewegung wird die Flüssigkeit in der Spitze des Proberöhrchens gesammelt und für 1 min in einem Heizblock bei 95 °C erhitzt. Mit einer Pasteurpipette wird unmittelbar die 95 °C heiße Flüssigkeit entnommen und 1 Tropfen auf die mikrofluidische Vorrichtung aufgegeben. Nach einer Inkubationszeit von 1 min werden 20 µl Enzymlösung in die Aufgabeöffnung der Plattform getropft. Nachdem diese eingesogen ist (ca. 30 s), werden 20 µl Waschlösung aufgegeben und wieder bis zum vollständigen Einsaugen der Flüssigkeit gewartet. Zuletzt wird die Farbreaktionslösung aufgegeben und die Farbreaktion bis zur optimalen Sichtbarkeit der Banden abgewartet. Die Reaktion ist nach ca. 2 min abgeschlossen und das Analyseergebnis wird visuell abgelesen, sowie mit einem Desktopscanner eingescannt.

Das Ausführungsbeispiel zeigt eine mögliche Ausführungsvariante des erfindungsgemäßen Verfahrens, wobei an dieser Stelle bemerkt sei, dass die Erfindung nicht auf die speziell angeführte Ausführungsvariante derselben eingeschränkt ist, sondern vielmehr durch die Patentansprüche definiert wird.

## Patentansprüche

1. Verfahren zum Nachweis und/oder zur Bestimmung von mit Parodontitis assoziierten Bakterien aus einer biologischen Probe umfassend die Schritte i) Gewinnung der biologischen Probe, ii) Denaturieren der biologischen Probe, insbesondere Zellen, iii) Hybridisierung der lysierten Probe mit zumindest einem der Oligonukleotide SEQ ID No. 4 oder SEQ ID No. 5 auf einem Träger und iv) Detektion des Ergebnisses.

2. Verfahren nach Anspruch 1, **dadurch gekennzeichnet, dass** der Nachweis und/oder die Bestimmung die Schritte a) Transfer der biologischen Probe in eine Universallösung enthaltend chaotrope Reagenzien und zumindest Oligonukleotid SEQ ID No. 6 in einen Behälter, b) Denaturieren der Probe, insbesondere durch Erhitzen des Behälters, c) Transfer zumindest eines Teils der Lösung in bzw. auf einen Träger mit zumindest einem immobilisierten Oligonukleotid der SEQ ID No. 5 und gegebenenfalls zusätzlich SEQ ID No. 7, 1 bis 3, und/oder 8, d) Hybridisierung und gegebenenfalls Inkubation und e) Detektion, umfasst.

3. Verfahren nach einem der Ansprüche 1 bis 2, **dadurch gekennzeichnet, dass** zusätzlich die Hybridisierung mit mindestens einer Sonde erfolgt, wobei die Sonde ausgewählt ist aus der Gruppe enthaltend: i) Oligonukleotid SEQ ID No. 1, 2, 3, 6, 7 oder 8, ii) Oligonukleotid, das ein gegenüber einem der Oligonukleotide SEQ ID No. 1, 2, 3, 6, 7 oder 8 mutierte Nukleotidsequenz aufweist, insbesondere eine Addition oder Deletion von 1 bis 10 Nukleotiden oder eine Substitution von 1 bis 3 Nukleotiden in einer der Nukleotidsequenzen, iii) Oligonukleotid, das eine Nukleotidsequenz aufweist, die zumindest bereichsweise zu der Nukleotidsequenz SEQ ID No. 1, 2, 3, 6, 7 oder 8 komplementär ist.

4. Verfahren nach einem der Ansprüche 1 bis 3, **dadurch gekennzeichnet, dass** als Träger ein Kapillarsystem, vorzugsweise ein Teststreifen oder eine mikrofluidische Vorrichtung, verwendet wird.

5. Verfahren nach einem der Ansprüche 1 oder 4, **dadurch gekennzeichnet; dass** das als Sonde verwendete Oligonukleotid ein DNA-, RNA-, PNA-, LNA-Molekül oder eine Mischform davon ist.

6. Verfahren nach einem der Ansprüche 1 bis 5, **dadurch gekennzeichnet, dass** in der Universallösung als chaotropes Reagenz Guanidiniumsalze, Guanidiniumisothiocyanat, Formamid, Harnstoff, Perchlorat, Thiocyanat, Trichloroacetat, Nitrat oder Iodid, vorzugsweise in einer Konzentration von 0,1 M bis 10 M, enthalten ist.

7. Verfahren nach einem der Ansprüche 1 bis 6, **dadurch gekennzeichnet, dass** in der Universallösung die Markierung der biologischen Probe, insbesondere durch das markierte Oligonukleotid SEQ ID No. 6, durchgeführt wird.

8. Verfahren nach einem der Ansprüche 1 bis 7, **dadurch gekennzeichnet, dass** die Hybridisierung bei Raumtemperatur durchgeführt wird.

9. Verfahren nach einem der Ansprüche 1 bis 8, **dadurch gekennzeichnet, dass** die Denaturierung der Probe bei einer Temperatur ausgewählt aus einem Bereich mit einer unteren Grenze von 50°C und einer oberen Grenze von 100°C durchgeführt wird.

10. Verfahren nach Anspruch 9, **dadurch gekennzeichnet, dass** der Transfer der vorzugsweise noch erhitzten biologischen Probe auf die mikrofluidische Vorrichtung mittels einer Pipette oder Kapillare erfolgt.

11. Verfahren nach einem der Ansprüche 1 bis 10, **dadurch gekennzeichnet, dass** die biologische Probe ohne vorab durchgeführte Amplifikation auf die mikrofluidische Vorrichtung aufgebracht wird.

12. Mikrofluidische Vorrichtung zum Nachweis und/oder zur Bestimmung zumindest eines mit Parodontitis assoziierten Keimes aus einer biologischen Probe umfassend einen Träger aus zumindest einem Bodenteil mit einer Oberfläche und mindestens ein an die Trägeroberfläche gebundenes Oligonukleotid oder Nukleinsäuremolekül, **dadurch gekennzeichnet, dass** zumindest eines der Oligonukleotide SEQ ID No. 4 oder SEQ ID No. 5 gebunden ist sowie optional mindestens ein weiteres Oligonukleotid, welches ausgewählt ist aus der Gruppe enthaltend: i) Oligonukleotid SEQ ID No. 1, 2, 3, 6, 7 oder 8, ii) Oligonukleotid, das ein gegenüber einem der Oligonukleotide SEQ ID No. 1, 2, 3, 6, 7 oder 8 mutierte Nukleotidsequenz aufweist, insbesondere eine Addition oder Deletion von 1 bis 10 Nukleotiden oder eine Substitution von 1 bis 3 Nukleotiden in einer der Nukleotidsequenzen, iii) Oligonukleotid, das eine Nukleotidsequenz aufweist, die zumindest bereichsweise zu der Nukleotidsequenz SEQ ID No. 1, 2, 3, 6, 7 oder 8 komplementär ist.

13. Mikrofluidische Vorrichtung nach Anspruch 12, **dadurch gekennzeichnet, dass** zumindest ein Kontrolloligonukleotid, vorzugsweise Positiv-, Negativ-, Orientierungs-, Hybri-disierungs-, Farbreaktionskontrolloligonukleotid, an der Trägeroberfläche angeordnet ist.

14. Kit zum Nachweis und/oder zur Bestimmung zumindest eines mit Parodontitis assoziierten Keimes aus einer biologischen Probe umfassend a) zumindest einen Träger, vorzugsweise eine mikrofluidische Vorrichtung, mit zumindest einem immobilisierten Oligonukleotid oder einer Nukleinsäuresequenz der SEQ ID No. 4 oder SEQ ID No. 5 sowie optional mindestens einem weiteren Oligonukleotid, welches ausgewählt ist aus der Gruppe enthaltend: i) Oligonukleotid SEQ ID No. 1, 2, 3, 6, 7 oder 8, ii) Oligonukleotid, das ein gegenüber einem der Oligonukleotide SEQ ID No. 1, 2, 3, 6, 7 oder 8 mutierte Nukleotidsequenz aufweist, insbesondere eine Addition oder Deletion von 1 bis 10 Nukleotiden oder eine Substitution von 1 bis 3 Nukleotiden in einer der Nukleotidsequenzen, iii) Oligonukleotid, das eine Nukleotidsequenz aufweist, die zumindest bereichsweise zu der Nukleotidsequenz SEQ ID No. 1, 2, 3, 6, 7 oder 8 komplementär ist; und b) zumindest einen Behälter mit einer Universallösung, welche zumindest ein chaotropes Reagenz sowie zumindest ein Oligonukleotid umfasst.

## Claims

1. Method for detecting and/or determining bacteria associated with periodontitis from a biological sample comprising the steps of i) recovering the biological sample, ii) denaturising the biological sample, in particular cells, iii) hybridising the lysated sample with at least one of the oligonucleotides SEQ ID No. 4 or SEQ ID No. 5 on a carrier and iv) detecting the result.

2. Method as claimed in claim 1, **characterised in that** the process of detection and/or determination comprises the steps of a) transferring the biological sample into a universal solution containing chaotropic reagents and at least oligonucleotide SEQ ID No. 6 in a container, b) denaturizing the sample, in particular by heating the container, c) transferring at least some of the solution into or onto a carrier with at least one immobilized oligonucleotide of SEQ ID No. 5 and optionally SEQ ID No. 7, 1 to 3, and/or 8, d) hybridization and optionally incubation, and e) detection.

3. Method as claimed in one of claims 1 or 2, **characterised in that** the hybridisation is additionally carried out with at least one probe, and the probe is selected from a group containing: i) oligonucleotide SEQ ID No. 1, 2, 3, 6, 7 or 8, ii) oligonucleotide which has a nucleotide sequence that is mutated in relation to one of the oligonucleotides SEQ ID No. 1, 2, 3, 6, 7 or 8, in particular an addition or deletion of 1 to 10 nucleotides or a substitution of 1 to 3 nucleotides in one of the nucleotide sequences, iii) oligonucleotide which has a nucleotide sequence which is complementary at least in areas to the nucleotide sequence SEQ ID No. 1, 2, 3, 6, 7 or 8.

4. Method as claimed in one of claims 1 to 3, **characterised in that** a capillary system is used as the carrier, preferably a test strip or microfluidic device.

5. Method as claimed in one of claims 1 or 4, **characterised in that** the oligonucleotide used as a probe is a DNA, RNA, PNA, LNA molecule or a mixed form thereof.

6. Method as claimed in one of claims 1 to 5, **characterised in that** the chaotropic reagent contained in the universal solution is guanidinium salts such as guanidinium-isothiocyanate, formamide, urea, perchlorate, thiocyanate, trichloroacetate, nitrate or iodide, preferably in a concentration of 0.1 M to 10 M.

7. Method as claimed in one of claims 1 to 6, **characterised in that** the biological sample is marked in the universal solution by the marked oligonucleotide SEQ ID No. 6 in particular.

8. Method as claimed in one of claims 1 to 7, **characterised in that** hybridisation takes place at room temperature.

9. Method as claimed in one of claims 1 to 8, **characterised in that** denaturisation of the sample takes place at a temperature selected from a range with a lower limit of 50°C and an upper limit of 100°C.

10. Method as claimed in claim 9, **characterised in that** the preferably still heated biological sample is transferred to the microfluidic device by means of a pipette or capillary.

11. Method as claimed in one of claims 1 to 10, **characterised in that** the biological sample is placed on the microfluidic device without prior amplification.

12. Microfluidic device for detecting and/or determining at least one germ associated with periodontitis from a biological sample, comprising a carrier consisting of at least one base part with a surface and at least one oligonucleotide or nucleic acid molecule bound onto the carrier surface, **characterised in that** at least one of the oligonucleotides SEQ ID No. 4 or SEQ ID No. 5 is bound and optionally at least one other oligonucleotide selected from the group containing: i) oligonucleotide SEQ ID No. 1, 2, 3, 6, 7 or 8, ii) oligonucleotide which has a nucleotide sequence that is mutated in relation to one of the oligonucleotides SEQ ID No. 1, 2, 3, 6, 7 or 8, in particular an addition or deletion of 1 to 10 nucleotides or a substitution of 1 to 3 nucleotides in one of the nucleotide sequences, iii) oligonucleotide which has a nucleotide sequence which is complementary at least in areas to the nucleotide sequence SEQ ID No. 1, 2, 3, 6, 7 or 8.

13. Microfluidic device as claimed in claim 12, **characterised in that** at least one control oligonucleotide is disposed on the carrier surface, preferably positive, negative, orientation, hybridization, colour reaction control oligonucleotide.

14. Kit for detecting and/or determining at least one germ associated with periodontitis from a biological sample, comprising a) at least one carrier, preferably a microfluidic device, with at least one immobilised oligonucleotide or nucleic acid sequence of SEQ ID No. 4 or SEQ ID No. 5 and optionally at least one other oligonucleotide selected from the group containing: i) oligonucleotide SEQ ID No. 1, 2, 3, 6, 7 or 8, ii) oligonucleotide which has a sequence which is mutated relative to one of the oligonucleotides SEQ ID No. 1, 2, 3, 6, 7 or 8, in particular an addition or deletion of 1 to 10 nucleotides or a substitution of 1 to 3 nucleotides in one of the nucleotide sequences, iii) oligonucleotide which is complementary at least in some areas to the nucleotide sequence SEQ ID No. 1, 2, 3, 6, 7 or 8, and b) at least one container with universal solution which contains at least one chaotropic reagent and at least one oligonucleotide.

## Revendications

1. Procédé de détection et/ou de détermination de bactéries associées à la parodontite depuis un échantillon biologique, comprenant les étapes i) obtention de l'échantillon biologique, ii) dénaturation de l'échantillon biologique, en particulier des cellules, iii) hybridation de l'échantillon lysé avec au moins un des oligonucléotides SEQ ID No. 4 ou SEQ ID No. 5 sur un support et iv) détection du résultat.

2. Procédé selon la revendication 1, **caractérisé en ce que** la détection et/ou la détermination comprend les étapes a) transfert de l'échantillon biologique dans une solution universelle contenant des réactifs chaotropiques et au moins l'oligonucléotide SEQ ID No. 6 dans un récipient, b) dénaturation de l'échantillon, en particulier par chauffage du récipient, c) transfert d'au moins une partie de la solution dans, respectivement sur un support avec au moins un oligonucléotide immobilisé du SEQ ID No. 5 et, le cas échéant, en plus du SEQ ID No. 7, 1 à 3, et/ou 8, d) hybridation et, le cas échéant, incubation et e) détection.

3. Procédé selon l'une quelconque des revendications 1 à 2, **caractérisé en ce qu'**en plus, l'hybridation s'effectue avec au moins une sonde, la sonde étant choisie parmi le groupe contenant i) un oligonucléotide SEQ ID No. 1, 2, 3, 6, 7 ou 8, ii) un oligonucléotide qui présente en comparaison d'un des oligonucléotides des SEQ ID No. 1, 2, 3, 6, 7 ou 8 des séquences de nucléotides mutées, en particulier une addition ou une délétion de 1 à 10 nucléotides ou une substitution de 1 à 3 nucléotides dans une des séquences de nucléotides, iii) un oligonucléotide qui présente une séquence de nucléotides qui est complémentaire au moins par tronçons de la séquence de nucléotides SEQ ID No. 1, 2, 3, 6, 7 ou 8.

4. Procédé selon l'une quelconque des revendications 1 à 3, **caractérisé en ce que** comme support est utilisé un système capillaire, de préférence une bande de contrôle ou un dispositif microfluidique.

5. Procédé selon l'une quelconque des revendications 1 ou 4, **caractérisé en ce que** l'oligonucléotide utilisé comme sonde est une molécule d'ADN, d'ARN, d'ANP, de LNA ou d'une forme mixte de ceux-ci.

6. Procédé selon l'une quelconque des revendications 1 à 5, **caractérisé en ce que** dans la solution universelle sont contenus comme réactifs chaotropiques des sels de guanidinium, de l'isothiocyanate de guanidinium, du formamide, de l'urée, du perchlorate, du thiocyanate, du trichloracétate, du nitrate ou de l'iodure, de préférence en une concentration de 0,1 M à 10 M.

7. Procédé selon l'une quelconque des revendications 1 à 6, **caractérisé en ce que** dans la solution universelle s'effectue le marquage des échantillons biologiques, en particulier par l'oligonucléotide marqué SEQ ID No. 6.

8. Procédé selon l'une quelconque des revendications 1 à 7, **caractérisé en ce que** l'hybridation est effectuée à la température ambiante.

9. Procédé selon l'une quelconque des revendications 1 à 8, **caractérisé en ce que** la dénaturation de l'échantillon est effectuée à une température choisie dans un intervalle avec une limite inférieure de 50 °C et une limite supérieure de 100 °C.

10. Procédé la revendication 9, **caractérisé en ce que** le transfert de l'échantillon biologique, de préférence encore chauffé, sur le dispositif microfluidique s'effectue au moyen d'une pipette ou d'un capillaire.

11. Procédé selon l'une quelconque des revendications 1 à 10, **caractérisé en ce que** l'échantillon biologique est appliqué sur le dispositif microfluidique sans qu'une amplification ait été effectuée auparavant.

12. Dispositif microfluidique pour la détection et/ou la détermination d'au moins un germe associé à la parodontite depuis un échantillon biologique, comprenant un support en au moins une pièce de fond avec une surface et au moins un oligonucléotide ou une molécule d'acide nucléique liés à la surface du support, **caractérisé en ce qu'**au moins un des oligonucléotides SEQ ID No. 4 ou SEQ ID No. 5 est lié, de même qu'en option au moins un autre oligonucléotide, lequel est choisi parmi le groupe contenant i) un oligonucléotide SEQ ID No. 1, 2, 3, 6, 7 ou 8, ii) un oligonucléotide qui présente en comparaison d'un des oligonucléotides des SEQ ID No. 1, 2, 3, 6, 7 ou 8 une séquence de nucléotides mutée, en particulier une addition ou une délétion de 1 à 10 nucléotides ou une substitution de 1 à 3 nucléotides dans une des séquences de nucléotides, iii) un oligonucléotide qui présente une séquence de nucléotides qui est complémentaire au moins par tronçons à la séquence de nucléotides SEQ ID No. 1, 2, 3, 6, 7 ou 8.

13. Dispositif microfluidique selon la revendication 12, **caractérisé en ce qu'**au moins un oligonucléotide de contrôle, de préférence un oligonucléotide de contrôle positif, négatif, d'orientation, d'hybridation, de réaction de couleur, est disposé à la surface du support.

14. Kit pour la détection et/ou la détermination d'au moins un germe associé à la parodontite depuis un échantillon biologique, comprenant a) au moins un support, de préférence un dispositif microfluidique avec au moins un oligonucléotide immobilisé ou une séquence d'acides nucléiques du SEQ ID No. 4 ou du SEQ ID No. 5, de même qu'en option au moins un autre oligonucléotide, lequel est choisi parmi le groupe contenant i) un oligonucléotide SEQ ID No. 1, 2, 3, 6, 7 ou 8, ii) un oligonucléotide qui présente en comparaison d'un des oligonucléotides des SEQ ID No. 1, 2, 3, 6, 7 ou 8 une séquence de nucléotides mutée, en particulier une addition ou une délétion de 1 à 10 nucléotides ou une substitution de 1 à 3 nucléotides dans une des séquences de nucléotides, iii) un oligonucléotide qui présente une séquence de nucléotides qui est complémentaire au moins par tronçons de la séquence de nucléotides SEQ ID No. 1, 2, 3, 6, 7 ou 8, et b) au moins un récipient avec une solution universelle, laquelle comporte au moins un réactif chaotropique ainsi qu'au moins un oligonucléotide.
